# EUROPEAN PATENT APPLICATION

(11) **EP 3 184 059 A1**
(43) Date of publication of application: **28.06.2017**
(21) Application number: 16840325.1
(22) Date of filing: 04.02.2016
(51) Int. Cl.: A61B 17/32

(54) **MEDICAL DEVICE**

(30) Priority: 16.09.2015 JP 2015183167
(71) Applicant: OLYMPUS CORPORATION, Shibuya-ku Tokyo 151-0072 (JP)
(72) Inventor: FU, Yuchun, Tokyo 151-0072 (JP); IGARASHI, Makoto, (JP)
(74) Representative: Schicker, Silvia
(86) International application number: PCT/JP2016/053327
(87) International publication number: WO 2017/047114

(57) **Abstract**

A medical device includes: an insertion portion formed in an elongated shape that can be inserted into an insertion hole of an insertion assisting instrument that assists insertion into a body cavity of a subject; a light guiding member configured to guide a light supplied from a light source to a distal end portion of the insertion portion; and an interlock mechanism provided in the insertion portion and configured to, in conjunction with an insertion state of the insertion portion with respect to the insertion hole, transition to either one of a state in which emission of a light to a front of the distal end portion of the insertion portion is possible and a state in which emission of a light to the front of the distal end portion of the insertion portion is not possible.

## Description

### Technical Field

The present invention relates to a medical device, and more particularly to a medical device that is used in a surgical operation at a site to be examined inside a body cavity.

### Background Art

In a medical field, for example, a surgical operation at a site to be examined inside a body cavity is sometimes performed while using a light having a high energy intensity (hereunder, also referred to as "high-energy light"), such as a laser beam.

Specifically, for example, Japanese Patent Application Laid-Open Publication No. 2010-82041 discloses an electronic endoscope system having a configuration which acquires a fluorescence image by administering a fluorescence reagent such as ICG (indocyanine green) to a subject, irradiating a laser beam in the near infrared region as excitation light at living tissue inside the subject, and picking up an image of the living tissue that emits light in response to irradiation of the excitation light.

Further, Japanese Patent Application Laid-Open Publication No. 2010-82041 also discloses a configuration which turns off the power supply of a laser beam source when an amount of light detected by a photodetector disposed at a position at which light emitted from a distal end of a laparoscope or an esophagogastroduodenoscope does not reach exceeds a specified value, and turns on the power supply of the laser beam source when an amount of light detected by the photodetector is less than or equal to the specified value.

In this connection, when performing a surgical operation at a site to be examined in a body cavity, for example, a situation can occur in which the operating room is darkened in order to facilitate viewing of an image or the like displayed on a display apparatus such as a monitor, and/or to psychologically calm the subject.

However, according to the configuration disclosed in Japanese Patent Application Laid-Open Publication No. 2010-82041, due the power supply of the laser beam source being turned on when the room in which the scope is disposed is darkened, the problem arises that, regardless of the fact that the scope is outside the subject, a laser beam that is generated from the laser beam source is emitted from the distal end of the scope.

Consequently, according to the configuration disclosed in Japanese Patent Application Laid-Open Publication No. 2010-82041, the issue arises that, because of the aforementioned problem, safety cannot be adequately ensured when performing a surgical operation at a site to be examined in a body cavity while using high-energy light.

The present invention has been made in consideration of the above described circumstances, and an object of the present invention is to provide a medical device that can adequately ensure safety when performing a surgical operation at a site to be examined in a body cavity while using high-energy light.

### Disclosure of Invention

### Means for Solving the Problem

A medical device according to one aspect of the present invention includes: an insertion portion formed in an elongated shape that can be inserted into an insertion hole of an insertion assisting instrument that assists insertion into a body cavity of a subject; a light guiding member configured to guide a light that is supplied from a light source to a distal end portion of the insertion portion; and an interlock mechanism that is provided in the insertion portion and is configured to, in conjunction with an insertion state of the insertion portion with respect to the insertion hole, transition to either one of a state in which emission of a light to a front of the distal end portion of the insertion portion is possible and a state in which emission of a light to the front of the distal end portion of the insertion portion is not possible.

### Brief Description of the Drawings

Fig. 1 is a view illustrating the configuration of a main portion of a medical system having a medical device according to an embodiment of the present invention;
Fig. 2 is a view for describing the configuration of an insertion portion of a medical device according to a first embodiment;
Fig. 3 is a view illustrating an example of a state in which the insertion portion of the medical device according to the first embodiment is inserted through a trocar;
Fig. 4 is a view for describing the configuration of an insertion portion of a medical device according to a second embodiment;
Fig. 5 is a view illustrating an example of a state in which the insertion portion of the medical device according to the second embodiment is inserted through a trocar;
Fig. 6 is a view for describing the configuration of an insertion portion of a medical device according to a third embodiment; and
Fig. 7 is a view illustrating an example of a state in which the insertion portion of the medical device according to the third embodiment is inserted through a trocar.

### Best Mode for Carrying Out the Invention

Hereunder, embodiments of the present invention are described with reference to the accompanying drawings.

### (First Embodiment)

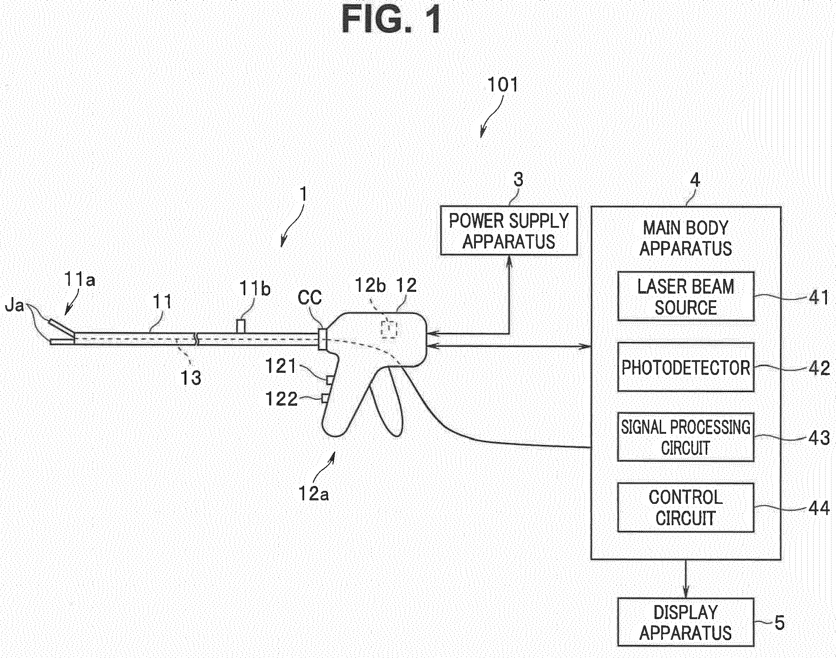

Fig. 1 to Fig. 3 relate to a first embodiment of the present invention.

As shown in Fig. 1, a medical system 101 includes a medical device 1, a power supply apparatus 3, a main body apparatus 4 and a display apparatus 5. Fig. 1 is a view that illustrates the configuration of a main portion of a medical system having a medical device relating to the embodiment.

The medical device 1 is constructed as a treatment instrument that can be inserted, for example, into a body cavity of a subject, and in accompaniment therewith can perform treatment by imparting energy that is in accordance with electric power supplied from the power supply apparatus 3 to a site to be examined such as living tissue in the body cavity of the subject. Specifically, the medical device 1 is constituted by, for example, a bipolar electric scalpel or a high-frequency treatment instrument. Further, the medical device 1 has, for example, as shown in Fig. 1, an insertion portion 11 and an operation portion 12. An optical fiber 13 for guiding an illuminating light that is supplied from the main body apparatus 4 and a return light that is generated in accordance with irradiation of the illuminating light, respectively, is inserted through and disposed inside the insertion portion 11 and the operation portion 12.

The insertion portion 11 is formed in an elongated shape that can be inserted into a body cavity of the subject through a trocar that is disposed in a body wall of the subject, which is, for example, a cylindrical shape. That is, the insertion portion 11 is formed in an elongated shape that can be inserted into an insertion hole of a trocar that is an insertion assisting instrument that assists insertion of the medical device 1 into the body cavity of the subject. Further, a treatment portion 11a for imparting energy in accordance with power that is supplied from the power supply apparatus 3 is provided at a distal end portion of the insertion portion 11. On a side face of the insertion portion 11, a protrusion portion 11b is provided that is formed so as to protrude in an external diameter direction that is a direction which is orthogonal to the longitudinal direction of the insertion portion 11. The insertion portion 11 is connected to the operation portion 12 through a connection connector CC that is provided at a proximal end portion. Further, in the insertion portion 11, an interlock mechanism is provided that is configured to, in conjunction with an insertion state with respect to an insertion hole of the trocar, perform an operation for transitioning to either one of a state in which emission of an illuminating light to the front of the distal end portion of the insertion portion 11 is possible and a state in which emission of the illuminating light to the front of the distal end portion of the insertion portion 11 is not possible. Specifically, the interlock mechanism of the insertion portion 11 includes the protrusion portion 11b, a switch portion that includes a cylinder portion 11d and a push switch 11f, and an elastic mechanism 11e.

The treatment portion 11a includes a pair of jaws Ja that are opened and closed in accordance with an operation of the operation portion 12, and is configured to be capable of imparting energy to living tissue or the like that is grasped by the pair of jaws Ja, as illustrated, for example, in Fig. 1.

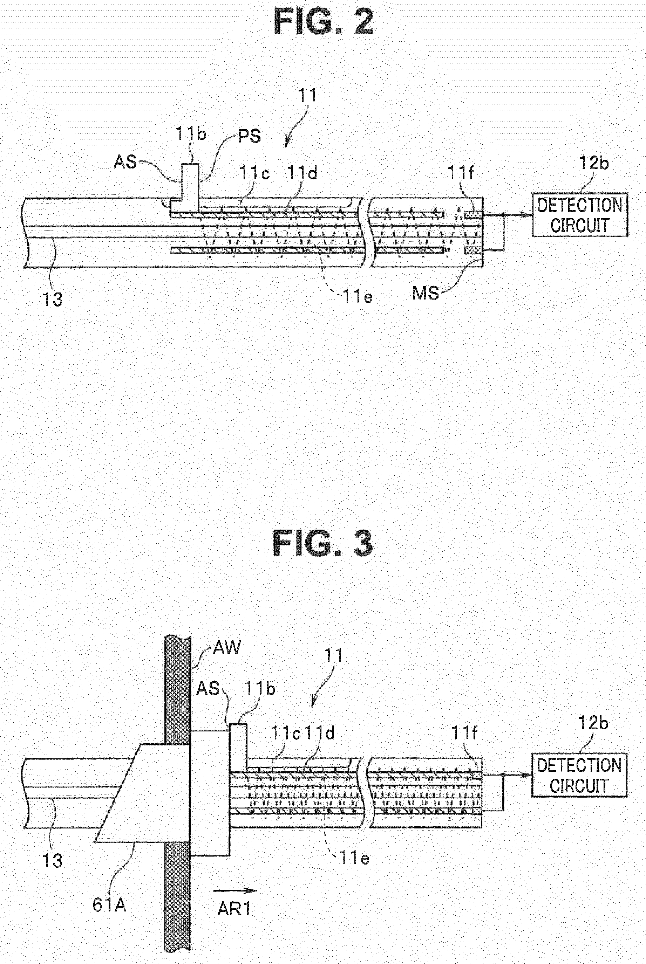

The protrusion portion 11b is formed, for example, so as to contact a surface on an entrance side of a trocar when the insertion portion 11 is inserted by an amount corresponding to a predetermined length through an insertion hole of the trocar. Further, for example, as shown in Fig. 2, the protrusion portion 11b is configured to be capable of sliding along a linear-shaped groove portion 11c provided in a parallel direction to the longitudinal direction on the side face of the insertion portion 11 in accordance with an external force applied to an action surface AS thereof that is a surface on a distal end side of the insertion portion 11. Further, as shown in Fig. 2, the protrusion portion 11b is formed integrally with the cylinder portion 11d inside the insertion portion 11. Fig. 2 is a view for describing the configuration of the insertion portion of the medical device according to the first embodiment.

The cylinder portion 11d, the elastic mechanism 11e and the push switch 11f are provided inside the insertion portion 11.

For example, as shown in Fig. 2, the cylinder portion 11d is formed in a hollow cylindrical shape through which the optical fiber 13 can be inserted, and is formed integrally with the protrusion portion 11b inside the insertion portion 11. That is, the cylinder portion 11d is configured as a tubular movable member configured to move in a parallel direction to the longitudinal direction of the insertion portion 11 in conjunction with movement of the protrusion portion 11b. Further, for example, as shown in Fig. 2, the cylinder portion 11d is formed so that, in a state in which an external force is not being applied to the action surface AS, the distal end portion thereof is disposed in the vicinity of the protrusion portion 11b and the proximal end portion thereof is disposed in the vicinity of the push switch 11f (described later).

The elastic mechanism 11e is formed, for example, by a helical compression spring that contracts in response to an external force. Further, for example, as shown in Fig. 2, the elastic mechanism 11e is disposed at a position that surrounds the outer circumferential face of the cylinder portion 11d along the longitudinal direction of the insertion portion 11. In addition, for example, as shown in Fig. 2, the elastic mechanism 11e is configured to have a length such that the elastic mechanism 11e is capable of connecting a surface PS that is a surface that is different to the action surface AS of the protrusion portion 11b and a proximal end surface MS that is a surface that is located at a position that is furthest on the proximal end side inside the insertion portion 11. Furthermore, the elastic mechanism 11e is configured to generate an urging force that urges the protrusion portion 11b to the distal end side of the insertion portion 11. Specifically, the elastic mechanism 11e is configured, for example, to generate an urging force which can cause the protrusion portion 11b to be disposed at a predetermined position that is furthest on the distal end side (hereunder, also referred to as "initial position") of the groove portion 11c in a state in which an external force is not being applied to the action surface AS. Note that, as long as the elastic mechanism 11e is formed so as to generate an urging force that urges the protrusion portion 11b to the distal end side of the insertion portion 11, the elastic mechanism 11e may be formed in another form that is different to a form that has a helical compression spring.

The push switch 11f is formed, for example, by a push-button switch, and is configured to enter an on state when pressed, and to enter an off state when not being pressed. Further, for example, as shown in Fig. 2, the push switch 11f is provided at a position facing the proximal end portion of the cylinder portion 11d on the proximal end surface MS, and is formed in a hollow cylindrical shape or an annular shape inside which the optical fiber 13 can be inserted.

That is, according to the configuration of the insertion portion 11 that is described above, for example, when an external force that exceeds the urging force produced by the elastic mechanism 11e is applied to the action surface AS, the protrusion portion 11b moves (slides) along the groove portion 11c from the initial position toward the proximal end side of the insertion portion 11, and the cylinder portion 11d also moves to the proximal end side of the insertion portion 11 in conjunction with movement of protrusion portion 11b. Further, according to the configuration of the insertion portion 11 that is described above, because the state of a pressing force on the push switch 11f changes depending on the position of the proximal end portion of the cylinder portion 11d that moves in conjunction with movement of the protrusion portion 11b, the push switch 11f can be changed to either of an "on" state that is a state in which it is possible to emit light to the front of the distal end portion of the insertion portion 11 and an "off" state that is a state in which it is not possible to emit light to the front of the distal end portion of the insertion portion 11.

The operation portion 12 is connected through the connection connector CC to the insertion portion 11. Further, for example, as shown in Fig. 1, the operation portion 12 has a handle portion 12a which can be utilized to perform operations relating to opening and closing of the pair of jaws Ja of the treatment portion 11a. A detection circuit 12b is also provided inside the operation portion 12.

A treatment switch 121 with which operations for turning the power supply from the power supply apparatus 3 on or off can be performed, and an illumination switch 122 with which operations for turning on or off the supply of an illuminating light from the main body apparatus 4 can be performed are provided on the handle portion 12a.

The detection circuit 12b is configured to be capable of detecting "on" and "off" states of the push switch 11f and the illumination switch 122, respectively. The detection circuit 12b is also configured to be capable of generating a state detection signal that indicates a result of detecting an "on" or "off" state of the push switch 11f and the illumination switch 122, and outputting the generated state detection signal to the main body apparatus 4.

A proximal end portion of the optical fiber 13 is provided in an extending manner from the proximal end side of the operation portion 12, and is configured to be detachably connected to the main body apparatus 4. A distal end portion of the optical fiber 13 is disposed in the vicinity of the pair of jaws Ja of the treatment portion 11a. Further, in the vicinity of an end face at a distal end portion of the optical fiber 13, a condenser lens (not shown) is provided for condensing an illuminating light that is emitted via the end face, and for also condensing a return light that is generated in accordance with irradiation of the illuminating light and for causing the condensed return light to be incident on the end face. That is, the optical fiber 13 is configured as a light guiding member configured to guide an illuminating light which is supplied from a laser beam source 41 (described later) of the main body apparatus 4 to the distal end portion of the insertion portion 11.

The power supply apparatus 3 is configured to include, for example, a high-frequency power supply. The power supply apparatus 3 is also configured to supply power to the medical device 1 that is connected thereto, when it is detected that the treatment switch 121 of the handle portion 12a is turned on. Furthermore, the power supply apparatus 3 is configured to stop the supply of power to the medical device 1 that is connected thereto, when it is detected that the treatment switch 121 of the handle portion 12a is turned off.

The main body apparatus 4 is configured to include the laser beam source 41, a photodetector 42, a signal processing circuit 43 and a control circuit 44.

The laser beam source 41 includes, for example, a laser diode, and is configured to generate a laser beam as an illuminating light that is irradiated onto a site to be examined that is to be treated using the medical device 1. The laser beam source 41 is also configured to supply a laser beam to the optical fiber 13 that is connected to the main body apparatus 4, when the laser beam source 41 is turned on in accordance with control of the control circuit 44. Further, the laser beam source 41 is configured to stop the supply of a laser beam to the optical fiber 13 that is connected to the main body apparatus 4, when the laser beam source 41 is turned off in accordance with control of the control circuit 44.

The photodetector 42 is equipped with, for example, an avalanche photodiode, and is configured to detect a return light that is emitted via the optical fiber 13 which is connected to the main body apparatus 4, and to generate and output a light detection signal in accordance with the intensity of the detected return light.

The signal processing circuit 43 is configured to, for example, generate and output an image in which the state of a site to be examined that is to be treated using the medical device 1 is visualized, by performing predetermined signal processing on the light detection signal outputted from the photodetector 42.

The control circuit 44 is configured to be capable of performing control relating to the operations of each portion of the main body apparatus 4. Specifically, the control circuit 44 is configured to, for example, based on a state detection signal outputted from the detection circuit 12b of the medical device 1 connected to the main body apparatus 4, perform control for turning on the laser beam source 41 in a case where both of the push switch 11f and the illumination switch 122 are in an "on" state, and to perform control for turning off the laser beam source 41 in a case where at least one of the push switch 11f and the illumination switch 122 is in an "off" state.

The display apparatus 5 includes, for example, a liquid crystal display, and is configured to be capable of displaying an image or the like that is outputted from the main body apparatus 4.

Next, the action of the present embodiment is described.

After a user such as a surgeon connects the respective portions of the medical system 101 and turns on the power, for example, the user distends the abdominal cavity of a subject by means of pneumoperitoneum gas that is supplied from a pneumoperitoneum apparatus (not shown), and in a state in which a trocar 61A is inserted in an abdominal wall AW of the subject, inserts the insertion portion 11 from an opening on the entrance side of an insertion hole of the trocar 61A.

According to the aforementioned operation of the user, for example, as shown in Fig. 3, accompanying insertion of the insertion portion 11 into the trocar 61A, because the action surface AS of the protrusion portion 11b is pressed against in a state in which the action surface AS contacts the surface on the entrance side of the trocar 61A that is inserted in the abdominal wall AW, a pressing force arises in the direction of an arrow AR1 that is a direction parallel or substantially parallel to the longitudinal direction of the insertion portion 11. Subsequently, as a result of a pressing force that exceeds the urging force generated by the elastic mechanism 11e being continuously applied to the action surface AS, the protrusion portion 11b and the cylinder portion 11d move toward the proximal end side of the insertion portion 11, and the proximal end portion of the cylinder portion 11d moves as far as a position at which the proximal end portion of the cylinder portion 11d presses the push switch 11f, and the push switch 11f thereby switches from an "off" state to an "on" state. Fig. 3 is a view that illustrates an example of a state in which the insertion portion of the medical device according to the first embodiment is inserted through the trocar.

That is, the cylinder portion 11d and the push switch 11f of the present embodiment maintain an "off" state in a case where a pressing force that exceeds an urging force produced by the elastic mechanism 11e is not being applied to the action surface AS of the protrusion portion 11b, and switch from the "off" state to an "on" state when the pressing force is applied to the action surface AS.

After inserting and disposing the insertion portion 11 inside the trocar 61 A, the user starts treatment at a site to be examined inside the abdominal cavity of the subject by performing operations to turn on the treatment switch 121 and the illumination switch 122 in a state in which the treatment portion 11a is caused to protrude from an opening on the exit side of the trocar 61A.

The detection circuit 12b detects an "on" or "off" state of the push switch 11f and the illumination switch 122, respectively, and when the push switch 11f and the illumination switch 122 are both in an "on" state the detection circuit 12b generates a state detection signal indicating a detection result to that effect, and outputs the state detection signal to the main body apparatus 4. In response to such operations of the detection circuit 12b, control to turn on the laser beam source 41 is performed by the control circuit 44, and the supply of a laser beam to the optical fiber 13 from the laser beam source 41 is started.

On the other hand, after performing an operation to turn off the treatment switch 121 to thereby end treatment at the site to be examined in the abdominal cavity of the subject, the user withdraws the insertion portion 11 from the trocar 61A.

In this case, according to the above described operation by the user, because the action surface AS is disposed at a position that is separated from the surface on the entrance side of the trocar 61A accompanying the withdrawal of the insertion portion 11 from the trocar 61A, the pressing force that was being applied to the action surface AS is removed. Further, as a result of the removal of the pressing force that was being applied to the action surface AS, the protrusion portion 11b and the cylinder portion 11d move to the distal end side of the insertion portion 11 in response to the urging force from the elastic mechanism 11e, and the proximal end portion of the cylinder portion 11d thus moves to a position that is separated from the push switch 11f, and the push switch 11f thereby switches to an "off" state from the "on" state.

That is, in conjunction with a contact state between the trocar 61A and the protrusion portion 11b, the cylinder portion 11d and the push switch 11f of the present embodiment perform operations for transitioning to either an "on" state that is a state in which light can be emitted to the front of the distal end portion of the insertion portion 11 or an "off" state that is a state in which light cannot be emitted to the front of the distal end portion of the insertion portion 11.

The detection circuit 12b detects an "on" or "off" state of the push switch 11f and the illumination switch 122, respectively, and when the push switch 11f is in an "off" state and the illumination switch 122 is in an "on" state the detection circuit 12b generates a state detection signal indicating a detection result to that effect, and outputs the state detection signal to the main body apparatus 4. In response to such operations of the detection circuit 12b, control to turn off the laser beam source 41 is performed by the control circuit 44, and the supply of a laser beam to the optical fiber 13 from the laser beam source 41 is stopped.

As described in the foregoing, according to the present embodiment, for example, even in a case where a situation arises in which the insertion portion 11 is withdrawn from the trocar 61A and is placed outside the body of the subject while the illumination switch 122 remains turned on, the supply of a laser beam from the laser beam source 41 to the optical fiber 13 can be stopped, that is, emission of a laser beam from the distal end portion of the insertion portion 11 can be prevented from occurring. Thus, according to the present embodiment, safety can be adequately ensured when performing a surgical operation at a site to be examined in a body cavity while using high-energy light.

Note that, by appropriately transforming the configuration of respective portions of the present embodiment, for example, a configuration may also be adopted so that, when the action surface AS contacts against a desired object such as the surface on the entrance side of the trocar 61 A, the cylinder portion 11d moves to the proximal end side of the insertion portion 11 and the push switch 11f thereby enters an "on" state, and in a case where the action surface AS is not contacting against the desired object, the cylinder portion 11d moves to the distal end side of the insertion portion 11 and the push switch 11f thereby enters an "off" state.

Further, the present embodiment is not limited to a configuration in which the control circuit 44 performs control for switching the laser beam source 41 between "on" and "off" states based on a state detection signal outputted from the detection circuit 12b, and for example a configuration may also be adopted in which the detection circuit 12b performs operations for switching the laser beam source 41 between "on" and "off" states based on a detection result regarding detection of an "on" or "off" state of the push switch 11f and the illumination switch 122, respectively.

Further, according to the present embodiment, for example, in a case where an optical fiber for illumination that is used for guiding illuminating light supplied from the main body apparatus 4 to the distal end portion of the insertion portion 11, and an optical fiber for receiving light that is used for guiding return light that is generated in accordance with irradiation of the illuminating light to the main body apparatus 4 from the distal end portion of the insertion portion 11 are provided instead of the optical fiber 13, the interlock mechanism may be configured so as to operate so that a state is entered in which the illuminating light that is guided by the optical fiber for illumination can be emitted to the front of the distal end portion of the insertion portion 11 or so that a state is entered in which the illuminating light cannot be emitted.

### (Second Embodiment)

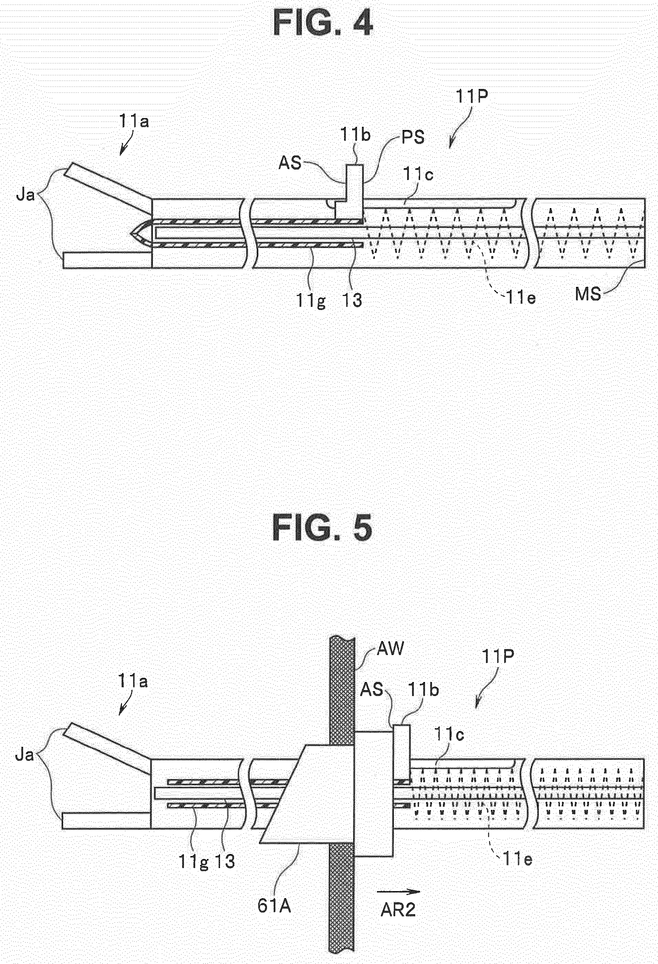

Fig. 4 and Fig. 5 relate to a second embodiment of the present invention.

Note that, in the present embodiment, a detailed description relating to portions having configurations or the like that are similar to configurations in the first embodiment will be omitted, and portions having configurations or the like that are different from configurations in the first embodiment will be mainly described.

Instead of the insertion portion 11 illustrated in Fig. 2, the medical device 1 of the present embodiment is provided with an insertion portion 11P that is illustrated, for example, in Fig. 4. Fig. 4 is a view for describing the configuration of the insertion portion of the medical device according to the second embodiment.

The insertion portion 11P has substantially the same configuration as the insertion portion 11, except that the insertion portion 11P is provided with a cylinder portion 11g instead of the cylinder portion 11d of the insertion portion 11, and the push switch 11f is removed relative to the configuration of the insertion portion 11. Further, the interlock mechanism of the insertion portion 11P is configured to include the protrusion portion 11b, the elastic mechanism 11e and the cylinder portion 11g.

The elastic mechanism 11e of the insertion portion 11P is disposed at a position that substantially faces the cylinder portion 11g along the longitudinal direction of the insertion portion 11.

The cylinder portion 11g is formed of a material that has a low thermal conductivity, such as silicone or fluorocarbon resin. Further, for example, as shown in Fig. 4, the cylinder portion 11g is formed in a hollow cylindrical shape through which the optical fiber 13 can be inserted, and is formed integrally with the protrusion portion 11b inside the insertion portion 11. That is, the cylinder portion 11g is configured as a tubular movable member configured to move in a parallel direction to the longitudinal direction of the insertion portion 11 in conjunction with movement of the protrusion portion 11b. Further, for example, as shown in Fig. 4, the cylinder portion 11g is formed so that, in a state in which an external force is not being applied to the action surface AS, the distal end portion thereof is disposed between the pair of jaws Ja, and the proximal end portion thereof is disposed in the vicinity of the protrusion portion 11b.

The distal end portion of the cylinder portion 11g is configured so that in a case where an external force is not being applied to the action surface AS, the distal end portion of the cylinder portion 11g maintains a closed state that blocks off a laser beam that is emitted via the end face of the distal end portion of the optical fiber 13, by, for example, as shown in Fig. 4, closing by forming a predetermined shape such as a dome shape. Furthermore, the distal end portion of the cylinder portion 11g is configured so as to transition to an open state that allows a laser beam emitted via the end face of the distal end portion of the optical fiber 13 to pass through by transforming to a shape that can be housed within the insertion portion 11 in response to application of an external force to the action surface AS.

An absorbent is coated on the inner circumferential face of the distal end portion of the cylinder portion 11g to, in a closed state, absorb a laser beam that is emitted via the end face of the distal end portion of the optical fiber 13. Further, on the outer circumferential face of the cylinder portion 11g, a conduit (not shown) is provided that is capable of circulating a coolant such as water for removing heat that is generated in response to absorption of a laser beam in a closed state.

That is, according to the configuration of the insertion portion 11P that is described above, for example, when an external force that exceeds the urging force produced by the elastic mechanism 11e is applied to the action surface AS, the protrusion portion 11b moves (slides) along the groove portion 11c from the initial position toward the proximal end side of the insertion portion 11, and the cylinder portion 11g also moves to the proximal end side of the insertion portion 11 in conjunction with movement of protrusion portion 11b. Further, according to the configuration of the insertion portion 11P that is described above, since the shape of the distal end portion of the cylinder portion 11g that moves in conjunction with movement of the protrusion portion 11b changes, the distal end portion of the cylinder portion 11 g can transition to either of an open state which allows light that is emitted via the optical fiber 13 to pass through or a closed state which blocks light that is emitted via the optical fiber 13.

The detection circuit 12b of the present embodiment is configured to detect "on" and "off" states of the illumination switch 122. Further, the detection circuit 12b of the present embodiment is configured to generate a state detection signal that indicates a result of detecting an "on" or "off" state of the illumination switch 122, and output the generated state detection signal to the main body apparatus 4.

The control circuit 44 of the present embodiment is configured to perform control for turning on the laser beam source 41 in a case where the illumination switch 122 is in an "on" state and to perform control for turning off the laser beam source 41 in a case where the illumination switch 122 is in an "off" state, based on a state detection signal outputted from the detection circuit 12b of the medical device 1 connected to the main body apparatus 4.

Next, the action of the present embodiment is described.

After a user such as a surgeon connects the respective portions of the medical system 101 and turns on the power, for example, the user distends the abdominal cavity of a subject by means of pneumoperitoneum gas that is supplied from a pneumoperitoneum apparatus (not shown), and in a state in which the trocar 61A is inserted in an abdominal wall AW of the subject, inserts the insertion portion 11P from an opening on the entrance side of the insertion hole of the trocar 61A.

According to the aforementioned operation of the user, for example, as shown in Fig. 5, accompanying insertion of the insertion portion 11P into the trocar 61A, because the action surface AS of the protrusion portion 11b is pressed against in a state in which the action surface AS contacts the surface on the entrance side of the trocar 61A that is inserted in the abdominal wall AW, a pressing force arises in the direction of an arrow AR2 that is a direction that is parallel or substantially parallel to the longitudinal direction of the insertion portion 11P. Subsequently, as a result of a pressing force that exceeds the urging force generated by the elastic mechanism 11e being continuously applied to the action surface AS, the protrusion portion 11b and the cylinder portion 11g move toward the proximal end side of the insertion portion 11, and the distal end portion of the cylinder portion 11g transitions from a closed state to an open state. Fig. 5 is a view that illustrates an example of a state in which the insertion portion of the medical device according to the second embodiment is inserted through the trocar.

That is, in a case where a pressing force that exceeds the urging force generated by the elastic mechanism 11e is not applied to the action surface AS of the protrusion portion 11b, the cylinder portion 11g of the present embodiment maintains a closed state by closing by forming a predetermined shape, and in a case where the pressing force is applied to the action surface AS, the cylinder portion 11g of the present embodiment transitions to an open state from the closed state by changing shape from the predetermined shape.

After inserting and disposing the insertion portion 11P inside the trocar 61 A, the user starts treatment at a site to be examined inside the abdominal cavity of the subject by performing operations to turn on the treatment switch 121 and the illumination switch 122 in a state in which the treatment portion 11a is caused to protrude from an opening on the exit side of the trocar 61A.

The detection circuit 12b detects an "on" or "off" state of the illumination switch 122, and when the illumination switch 122 is in an "on" state the detection circuit 12b generates a state detection signal indicating a detection result to that effect, and outputs the state detection signal to the main body apparatus 4. In response to such operations of the detection circuit 12b, control to turn on the laser beam source 41 is performed by the control circuit 44, and the supply of a laser beam to the optical fiber 13 from the laser beam source 41 is started.

On the other hand, after performing an operation to turn off the treatment switch 121 to thereby end treatment of the site to be examined in the abdominal cavity of the subject, the user withdraws the insertion portion 11P from the trocar 61A.

In this case, according to the above described operation by the user, because the action surface AS is disposed at a position that is separated from the surface on the entrance side of the trocar 61A accompanying the withdrawal of the insertion portion 11P from the trocar 61A, the pressing force that was being applied to the action surface AS is removed. Further, as a result of the removal of the pressing force that was being applied to the action surface AS, the protrusion portion 11b and the cylinder portion 11g move to the distal end side of the insertion portion 11P in response to the urging force of the elastic mechanism 11e, and the distal end portion of the cylinder portion 11g transitions from an open state to a closed state.

That is, in conjunction with a contact state between the trocar 61A and the protrusion portion 11b, the cylinder portion 11g of the present embodiment transitions to either one of an open state that allows light that is emitted via the optical fiber 13 to pass through and a closed state that blocks light that is emitted via the optical fiber 13.

As described in the foregoing, according to the present embodiment, for example, even in a case where a situation arises in which the insertion portion 11P is withdrawn from the trocar 61A and is placed outside the body of the subject while the illumination switch 122 remains turned on, because a laser beam that is emitted via the optical fiber 13 is blocked by the cylinder portion 11g that is in a closed state, emission of the laser beam from the distal end portion of the insertion portion 11P can be prevented from occurring. Therefore, according to the present embodiment, safety can be adequately ensured when performing a surgical operation at a site to be examined in a body cavity while using high-energy light.

### (Third Embodiment)

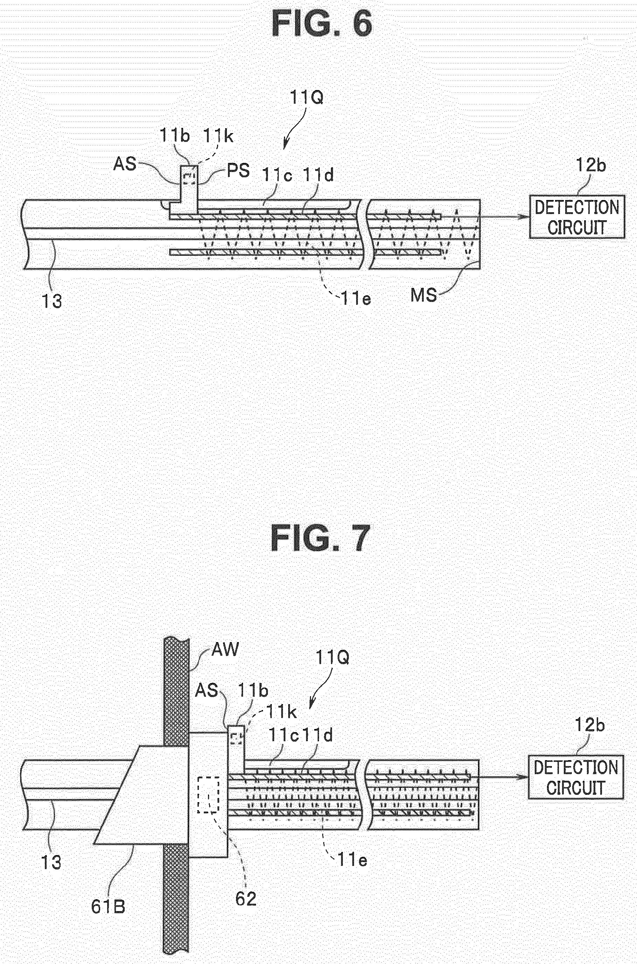

Fig. 6 and Fig. 7 relate to a third embodiment of the present invention.

Note that, in the present embodiment, a detailed description relating to portions having configurations or the like that are similar to configurations in at least one of the first and second embodiments will be omitted, and portions having configurations or the like that are different from configurations in both the first and second embodiments will be mainly described.

Instead of the insertion portion 11 illustrated in Fig. 2, the medical device 1 of the present embodiment includes an insertion portion 11 Q that is illustrated, for example, in Fig. 6. Fig. 6 is a view for describing the configuration of the insertion portion of the medical device according to the third embodiment.

The insertion portion 11 Q has substantially the same configuration as the insertion portion 11, except that the insertion portion 11Q is provided with a magnetic sensor 11k inside the protrusion portion 11b of the insertion portion 11, and the push switch 11f is removed relative to the configuration of the insertion portion 11. Further, the interlock mechanism of the insertion portion 11 Q is configured to include the protrusion portion 11b and the magnetic sensor 11k.

The magnetic sensor 11k, for example, is provided inside the protrusion portion 11b and is configured to detect a magnetic field in the vicinity of the action surface AS of the protrusion portion 11b, and generate a magnetic field detection signal having a signal level that is in accordance with the strength of the detected magnetic field. That is, the magnetic sensor 11k is configured to move in a parallel direction to the longitudinal direction of the insertion portion 11 in conjunction with movement of the protrusion portion 11b. Further, the magnetic sensor 11k is configured to output a magnetic field detection signal that is generated as described above to the detection circuit 12b through, for example, an unshown signal wire that is inserted through the inside of the protrusion portion 11b and the cylinder portion 11d.

The detection circuit 12b of the present embodiment is configured so as to detect the signal level of the magnetic field detection signal that is outputted from the magnetic sensor 11k, and an "on" or "off" state of the illumination switch 122, respectively. Further, the detection circuit 12b of the present embodiment is configured to generate a state detection signal indicating a detection result with respect to the signal level of the magnetic field detection signal outputted from the magnetic sensor 11k as well as the "on" or "off" state of the illumination switch 122, and to output the generated state detection signal to the main body apparatus 4.

The control circuit 44 of the present embodiment is configured to perform control for turning on the laser beam source 41 in a case where, based on the state detection signal outputted from the detection circuit 12b of the medical device 1 that is connected to the main body apparatus 4, the signal level of the magnetic field detection signal that is outputted from the magnetic sensor 11k is equal to or greater than a predetermined threshold value TH and the illumination switch 122 is in an "on" state. Further, the control circuit 44 of the present embodiment is configured to, based on the state detection signal outputted from the detection circuit 12b of the medical device 1 that is connected to the main body apparatus 4, perform control for turning off the laser beam source 41 in either of a case where the signal level of the magnetic field detection signal that is outputted from the magnetic sensor 11k is less than the predetermined threshold value TH or a case where the illumination switch 122 is in an "off" state.

Next, the action of the present embodiment is described.

After a user such as a surgeon connects the respective portions of the medical system 101 and turns on the power, for example, the user distends the abdominal cavity of a subject by means of pneumoperitoneum gas that is supplied from a pneumoperitoneum apparatus (not shown), and in a state in which a trocar 61B having a built-in magnet 62 that generates a magnetic field of a predetermined strength is inserted in an abdominal wall AW of the subject, inserts the insertion portion 11 Q from an opening on the entrance side of an insertion hole of the trocar 61B.

According to the aforementioned operation of the user, for example, as shown in Fig. 7, accompanying insertion of the insertion portion 11Q into the trocar 61B, the action surface AS of the protrusion portion 11b is pressed against in a state in which the action surface AS contacts the surface on the entrance side of the trocar 61B that is inserted in the abdominal wall AW, and the magnetic sensor 11k is disposed at a position at which the magnetic sensor 11k is capable of detecting a magnetic field that emanates from the magnet 62. Fig. 7 is a view that illustrates an example of a state in which the insertion portion of the medical device according to the third embodiment is inserted through the trocar.

The magnetic sensor 11k generates a magnetic field detection signal with a signal level SG1 that is equal to or greater than the predetermined threshold value TH as a magnetic field detection signal that is in accordance with the strength of the magnetic field emanating from the magnet 62, and outputs the magnetic field detection signal to the detection circuit 12b.

That is, in conjunction with a contact state between the trocar 61B and the protrusion portion 11b, when disposed at a position at which the magnetic field emanating from the magnet 62 can be detected, the magnetic sensor 11k of the present embodiment generates and outputs a magnetic field detection signal with the signal level SG1 that indicates a state in which light can be emitted to the front of the distal end portion of the insertion portion 11Q.

After inserting and disposing the insertion portion 11Q inside the trocar 61B, the user starts treatment at a site to be examined inside the abdominal cavity of the subject by performing operations to turn on the treatment switch 121 and the illumination switch 122 in a state in which the treatment portion 11a is caused to protrude from an opening on the exit side of the trocar 61B.

The detection circuit 12b detects the signal level of the magnetic field detection signal outputted from the magnetic sensor 11k and an "on" or "off" state of the illumination switch 122, respectively, and when the signal level of the magnetic field detection signal is SG1 and the illumination switch 122 is in an "on" state the detection circuit 12b generates a state detection signal indicating a detection result to that effect, and outputs the state detection signal to the main body apparatus 4. In response to such operations of the detection circuit 12b, control to turn on the laser beam source 41 is performed by the control circuit 44, and the supply of a laser beam to the optical fiber 13 from the laser beam source 41 is started.

On the other hand, after performing an operation to turn off the treatment switch 121 to thereby end treatment of the site to be examined in the abdominal cavity of the subject, the user withdraws the insertion portion 11Q from the trocar 61B.

According to the above described operation performed by the user, the action surface AS separates from the surface on the entrance side of the trocar 61B accompanying withdrawal of the insertion portion 11 from the trocar 61B, and the magnetic sensor 11k is thereby disposed at a position at which it is not possible to detect the magnetic field emanating from the magnet 62, and consequently the signal level of the magnetic field detection signal outputted from the magnetic sensor 11k decreases from SG1 to a signal level SG2 that is less than the predetermined threshold value TH.

That is, in conjunction with a contact state between the trocar 61B and the protrusion portion 11b, when disposed at a position at which the magnetic field emanating from the magnet 62 cannot be detected, the magnetic sensor 11k of the present embodiment generates and outputs a magnetic field detection signal with the signal level SG2 that indicates a state in which light cannot be emitted to the front of the distal end portion of the insertion portion 11Q.

The detection circuit 12b detects the signal level of the magnetic field detection signal outputted from the magnetic sensor 11k and an "on" or "off" state of the illumination switch 122, respectively, and when the signal level of the magnetic field detection signal is SG2 and the illumination switch 122 is in an "on" state the detection circuit 12b generates a state detection signal indicating a detection result to that effect, and outputs the state detection signal to the main body apparatus 4. In response to such operations of the detection circuit 12b, control to turn off the laser beam source 41 is performed by the control circuit 44, and the supply of a laser beam to the optical fiber 13 from the laser beam source 41 is stopped.

As described in the foregoing, according to the present embodiment, for example, even in a case where a situation arises in which the insertion portion 11Q is withdrawn from the trocar 61B and is placed outside the body of the subject while the illumination switch 122 remains turned on, the supply of a laser beam from the laser beam source 41 to the optical fiber 13 can be stopped, that is, emission of a laser beam from the distal end portion of the insertion portion 11 Q can be prevented from occurring. Thus, according to the present embodiment, safety can be adequately ensured when performing a surgical operation at a site to be examined in a body cavity while using high-energy light.

Note that, according to the present embodiment, a configuration may be adopted in which, instead of the magnetic sensor 11k, the insertion portion 11Q includes, on the action surface AS of the protrusion portion 11b, an optical sensor for detecting light emitted from a light emitting device which is provided, for example, on the surface on the entrance side of the trocar 61B. In accordance with such a configuration, the control circuit 44 may be configured to perform control to turn on the laser beam source 41 in a case where, for example, a signal level of a light detection signal that is outputted from the optical sensor of the protrusion portion 11b is equal to or greater than a predetermined threshold value THA and the illumination switch 122 is in an "on" state.

Further, according to the present embodiment, a configuration may be adopted in which, instead of the magnetic sensor 11k, the insertion portion 11Q includes, for example, on the action surface AS of the protrusion portion 11b, a force sensor for detecting a pressing force that arises as a result of contact with a desired object such as the surface on the entrance side of the trocar 61A (or 61B). In accordance with such a configuration, the control circuit 44 may be configured to perform control to turn on the laser beam source 41 in a case where, for example, a signal level of a force detection signal that is outputted from the force sensor of the protrusion portion 11b is equal to or greater than a predetermined threshold value THB and the illumination switch 122 is in an "on" state.

Furthermore, according to the present embodiment, a configuration may be adopted in which, instead of the magnetic sensor 11k, the insertion portion 11Q includes, for example, on the action surface AS of the protrusion portion 11b, a distance measuring sensor for measuring a distance between the action surface AS and a desired object such as the surface on the entrance side of the trocar 61A (or 61B). In accordance with such a configuration, the control circuit 44 may be configured to perform control to turn on the laser beam source 41 in a case where, for example, a distance measured by the distance measuring sensor of the protrusion portion 11b is 0 or approximately 0 and the illumination switch 122 is in an "on" state.

It should be understood that the present invention is not limited to the respective embodiments described above, and naturally various modifications and applications are possible without departing from the gist of the invention.

The present application is based upon and claims priority from Japanese Patent Application No. 2015-183167 filed in Japan on September 16, 2015, the contents of which are hereby incorporated by reference in their entirety into the description, claims and drawings of the present application.

## Claims

1. A medical device, comprising:
an insertion portion formed in an elongated shape that can be inserted into an insertion hole of an insertion assisting instrument that assists insertion into a body cavity of a subject;
a light guiding member configured to guide a light that is supplied from a light source to a distal end portion of the insertion portion; and
an interlock mechanism that is provided in the insertion portion and is configured to, in conjunction with an insertion state of the insertion portion with respect to the insertion hole, transition to either one of a state in which emission of a light to a front of the distal end portion of the insertion portion is possible and a state in which emission of a light to the front of the distal end portion of the insertion portion is not possible.

2. The medical device according to claim 1, wherein the interlock mechanism comprises:
a protrusion portion that is provided on a side face of the insertion portion and is formed so as to contact a surface of the insertion assisting instrument when the insertion portion is inserted by an amount corresponding to a predetermined length through the insertion hole; and
a switch portion configured to, in conjunction with a contact state between the insertion assisting instrument and the protrusion portion, perform an operation for transitioning to either one of an "on" state that is a state in which emission of a light to the front of the distal end portion of the insertion portion is possible and an "off" state that is a state in which emission of a light to the front of the distal end portion of the insertion portion is not possible.

3. The medical device according to claim 2, wherein:
the interlock mechanism further comprises an elastic mechanism configured to generate an urging force capable of causing the protrusion portion that is in a state of non-contact with respect to the insertion assisting instrument to be disposed at a predetermined position of the insertion portion;
the protrusion portion is formed so as to move from the predetermined position to a proximal end side of the insertion portion when an external force exceeding the urging force is applied in accordance with contact with the insertion assisting instrument; and
the switch portion is configured to maintain the "off" state in a case where the external force is not applied to the protrusion portion, and to switch from the "off" state to the "on" state in a case where the external force is applied to the protrusion portion.

4. The medical device according to claim 1, wherein the interlock mechanism comprises:
a protrusion portion that is provided on a side face of the insertion portion and is formed so as to contact a surface of the insertion assisting instrument when the insertion portion is inserted by an amount corresponding to a predetermined length through the insertion hole; and
a cylinder portion that is formed so as to, in conjunction with a contact state between the insertion assisting instrument and the protrusion portion, transition to either one of an open state that allows a light that is emitted via the light guiding member to pass through and a closed state that blocks a light that is emitted via the light guiding member.

5. The medical device according to claim 4, wherein:
the interlock mechanism further comprises an elastic mechanism configured to generate an urging force capable of causing the protrusion portion that is in a state of non-contact with respect to the insertion assisting instrument to be disposed at a predetermined position of the insertion portion;
the protrusion portion is formed so as to move from the predetermined position to a proximal end side of the insertion portion when an external force exceeding the urging force is applied in accordance with contact with the insertion assisting instrument; and
the cylinder portion is formed so as to maintain the closed state by closing by forming a predetermined shape in a case where the external force is not being applied to the protrusion portion, and to transition from the closed state to the open state by changing shape from the predetermined shape when the external force is being applied to the protrusion portion.

6. The medical device according to claim 1, wherein:
the interlock mechanism comprises a protrusion portion that is provided on a side face of the insertion portion and is formed so as to contact a surface of the insertion assisting instrument when the insertion portion is inserted by an amount corresponding to a predetermined length through the insertion hole, and a magnetic sensor configured to detect a magnetic field in a vicinity of the protrusion portion; and
the magnetic sensor is configured to, when disposed at a position at which the magnetic sensor can detect a magnetic field that emanates from a magnet provided in the insertion assisting instrument, output a magnetic field detection signal that indicates a state in which emission of a light to the front of the distal end portion of the insertion portion is possible.
